(19) Europäisches Patentamt
European Patent Office
Office européen des brevets

(11) Veröffentlichungsnummer : **0 144 648**
**B1**

(12) # EUROPÄISCHE PATENTSCHRIFT

(45) Veröffentlichungstag der Patentschrift : **21.10.87**

(51) Int. Cl.⁴ : **C 07 C 121/00**, C 07 C 69/753, C 09 K 19/30

(21) Anmeldenummer : **84112552.9**

(22) Anmeldetag : **18.10.84**

(54) **Flüssigkristalline Cyclohexylbenzol- und Cyclohexylbiphenylderivate.**

(30) Priorität : **02.11.83 CH 5912/83**
**20.08.84 CH 3970/84**

(43) Veröffentlichungstag der Anmeldung : **19.06.85 Patentblatt 85/25**

(45) Bekanntmachung des Hinweises auf die Patenterteilung : **21.10.87 Patentblatt 87/43**

(84) Benannte Vertragsstaaten :
**AT BE CH DE FR GB IT LI NL**

(56) Entgegenhaltungen :
**EP-A- 0 044 646**
**EP-A- 0 090 671**
**DE-A- 3 324 774**
**GB-A- 2 086 385**
**GB-A- 2 107 733**
**CHEMICAL ABSTRACTS, Band 100, Nr. 20, 14. Mai 1984, Seite 572, Nr. 165520r, Columbus, Ohio, US**

(73) Patentinhaber : **F. HOFFMANN-LA ROCHE & CO. Aktiengesellschaft**
**CH-4002 Basel (CH)**

(72) Erfinder : **Oesterhelt, Gottfried**
**Eggfluhstrasse 18**
**CH-4153 Reinach (CH)**
Erfinder : **Petrzilka, Martin, Dr.**
**Schwarzackerstrasse 54**
**CH-4303 Kaiseraugst (CH)**

(74) Vertreter : **Zimmermann, Hans, Dr. et al**
**Grenzacherstrasse 124 Postfach 3255**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 144 648 B1

Jouve, 18, rue St-Denis, 75001 Paris, France

**Beschreibung**

Die vorliegende Erfindung betrifft neue flüssigkristalline Verbindungen, nämlich die 4'-(trans-4-Alkylcyclohexyl)-4-biphenylyl-Derivate und 4-(trans-4-Alkylcyclohexyl) phenyl-Derivate der allgemeinen Formel

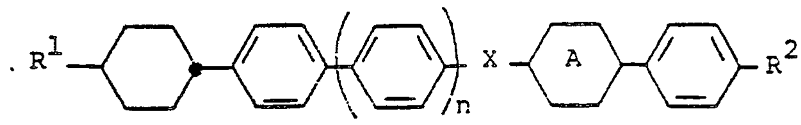
(I)

worin n für die Zahl 1 und X für die Gruppe —COO—, —COC— oder —$CH_2CH_2$— steht, oder n für die Zahl 0 und X für die Gruppe —$CH_2CH_2$— steht ; Ring A p-Phenylen oder trans-1,4-Cyclohexylen bezeichnet ; $R^2$ Cyano, p-Cyanophenyl oder, sofern X für —COO— oder —OOC— steht, auch p-$R^3$-Phenyl oder trans-4-$R^3$-Cyclohexyl darstellt ; und $R^1$ und $R^3$ geradkettiges $C_1$-$C_{10}$-Alkyl bedeuten.

Die Erfindung betrifft ferner die Herstellung der Verbindungen der obigen Formel I, flüssigkristalline Gemische, welche Verbindungen der obigen Formel I enthalten, sowie die Verwendung für elektrooptische und chromatographische Zwecke.

Flüssige Kristalle finden vor allem als Dielektrika in Anzeigevorrichtungen Verwendung, da sich die optischen Eigenschaften solcher Substanzen durch elektrische Felder beeinflussen lassen. Elektrooptische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Typ), dem Schadt-Helfrich-Effekt (Drehzelle), dem Gast/Wirt-Effekt (« Guest/-Host-Zelle ») oder einem cholesterisch-nematischen Phasenübergang.

Da es im allgemeinen nicht möglich ist, alle gewünschten Eigenschaften, wie beispielsweise hohe chemische und photochemische Stabilität, niedere Viskosität, grosse nematische bzw. cholesterische Mesophasenbereiche, kurze Ansprechzeiten und dergleichen, mit einer einzigen Verbindung zu erreichen, werden meist Mischungen mehrerer Komponenten verwendet. Derartige Mischungen enthalten in der Regel hauptsächlich niederviskose Verbindungen, welche aber anderseits meist auch niedrige Klärpunkte aufweisen. Aus diesem Grunde müssen den Mischungen oft klärpunktserhöhende Substanzen zugesetzt werden.

EP-A-0 044 646 offenbart 4-(trans-4-Alkylcyclohexyl)-benzoesäure-4'-cyano-4-biphenylylester als klärpunktserhöhende Zusätze für Flüssigkristallmischungen.

Flüssige Kristalle wurden bereits auch als stationäre Phasen in der Gaschromatographie eingesetzt. Die bisher bekannten, flüssigkristallinen, stationären Phasen haben aber im allgemeinen den Nachteil, dass sie nur bis zu Temperaturen von etwa 180 °C verwendet werden können.

Es wurde nun gefunden, dass die erfindungsgemässen Verbindungen sehr grosse Mesophasenbereiche mit hohen Klärpunkten ausbilden, und dass sie im allgemeinen im ganzen oder zumindest im überwiegenden Teil des flüssigkristallinen Bereichs eine nematische Mesophase aufweisen. Sie besitzen eine gute chemische und photochemische Stabilität und sind farblos. Die Viskositätswerte sind unter Berücksichtigung der hohen Klärpunkte vergleichsweise niedrig. Diejenigen Verbindungen der Formel I, worin $R^2$ Cyano oder p-Cyanophenyl bedeutet, besitzen eine positive dielektrische Anisotropie ; die übrigen Verbindungen der Formel I (d. h. diejenigen, worin $R^2$ p-Alkylphenyl oder trans-4-Alkylcyclohexyl bedeutet) besitzen kleine absolute Werte der dielektrischen Anisotropie.

Aufgrund ihrer sehr guten Trennfähigkeit und ihrer hohen Klärpunkte und Siedepunkte eignen sich die erfindungsgemässen Verbindungen vorzüglich für oder als stationäre Phasen in der Gaschromatographie und können auch bei sehr hohen Arbeitstemperaturen angewendet werden. Besonders geeignet sind die erfindungsgemässen Verbindungen zur Trennung von Isomerengemischen, welche mit konventionellen stationären Phasen nicht oder nur schlecht trennbar sind, wie beispielsweise cis/trans-isomere Cyclopentane und Cyclohexane (z. B. 1,4-disubstituierte Cyclohexane), isomere aromatische Kohlenwasserstoffe (z. B. o-, m- und p-disubstituierte Benzole, Anthracen/Phenanthren), Doppelbindungsisomere (z. B. ungesättigte Fettsäureester) und dergleichen. Die erfindungsgemässen Verbindungen eignen sich insbesondere auch zur Anwendung in Kapillarsäulen. Ferner eignen sich insbesondere die Verbindungen der Formel I, worin X die Gruppe —$CH_2CH_2$— bedeutet, auch zur Trennung von silylierten Proben. Derartige Proben von Silylderivaten (z. B. Trimethylsilylderivate) werden oft hergestellt, wenn Gemische von Verbindungen mit aciden Wasserstoffatomen (z. B. Säuren, Alkohole, Phenole, Amine, Amide) vorliegen, um die Trennung zu erleichtern.

Weiterhin sind die erfindungsgemässen Verbindungen als klärpunktserhöhende Substanzen für flüssigkristalline Dielektrika geeignet. Hierbei haben sie vor allem den Vorteil, dass entweder sehr grosse Klärpunktserhöhungen erreicht werden können oder anderseits zur Erzielung einer bestimmten Klärpunktserhöhung nur vergleichsweise geringe Mengen zugesetzt werden müssen und damit die übrigen Eigenschaften des Gemisches im allgemeinen nur unwesentlich verändert werden.

Die erfindungsgemässen Verbindungen sind tetracyclische, pentacyclische oder hexacyclische Verbindungen mit 1 oder 2 polaren Gruppen, nämlich einer Estergruppe und/oder einer Cyanogruppe.

Die Alkylreste $R^1$ und $R^3$ bedeuten unabhängig voneinander Methyl, Aethyl, Propyl, Butyl, Pentyl, Hexyl, Heptyl, Octyl, Nonyl und Decyl.

Rest $R^2$ in obiger Formel I bezeichnet vorzugsweise Cyano, p-Cyanophenyl oder p-$R^3$-Phenyl, wenn Ring A trans-1,4-Cyclohexylen bedeutet, und trans-4-$R^3$-Cyclohexyl, wenn Ring A p-Phenylen bedeutet. X steht vorzugsweise für die Gruppe —COO— oder —$CH_2CH_2$—. Die Alkylgruppen $R^1$ und $R^3$ bedeuten unabhängig voneinander vorzugsweise geradkettiges $C_1$-$C_7$-Alkyl und insbesondere Propyl, Butyl oder Pentyl. Vorzugsweise steht n für die Zahl 1.

Beispiele bevorzugter erfindungsgemässer Verbindungen sind die Verbindungen der obigen Formel I, worin $R^1$, X, Ring A und $R^2$ die in Tabelle 1 gegebenen Bedeutungen haben (—$C_6H_4$— bezeichnet p-Phenylen und —$C_6H_{10}$— bezeichnet trans-1,4-Cyclohexylen) und n für die Zahl 1 steht, sowie die weiteren in den Synthesebeispielen genannten Verbindungen der Formel I.

Tabelle 1

| $R^1$ | X | Ring A | $R^2$ |
|---|---|---|---|
| $C_3H_7$- | $-CH_2CH_2-$ | $-C_6H_{10}-$ | -CN |
| $C_4H_9$- | $-CH_2CH_2-$ | $-C_6H_{10}-$ | -CN |
| $C_5H_{11}$- | $-CH_2CH_2-$ | $-C_6H_{10}-$ | -CN |
| $C_3H_7$- | $-CH_2CH_2-$ | $-C_6H_{10}-$ | $-C_6H_4-CN$ |
| $C_4H_9$- | $-CH_2CH_2-$ | $-C_6H_{10}-$ | $-C_6H_4-CN$ |
| $C_5H_{11}$- | $-CH_2CH_2-$ | $-C_6H_{10}-$ | $-C_6H_4-CN$ |
| $C_3H_7$- | -COO- | $-C_6H_{10}-$ | -CN |
| $C_4H_9$- | -COO- | $-C_6H_{10}-$ | -CN |
| $C_5H_{11}$- | -COO- | $-C_6H_{10}-$ | -CN |
| $C_3H_7$- | -COO- | $-C_6H_{10}-$ | $-C_6H_4-CN$ |
| $C_4H_9$- | -COO- | $-C_6H_{10}-$ | $-C_6H_4-CN$ |
| $C_5H_{11}$- | -COO- | $-C_6H_{10}-$ | $-C_6H_4-CN$ |
| $C_3H_7$- | -COO- | $-C_6H_4-$ | -CN |
| $C_5H_{11}$- | -COO- | $-C_6H_4-$ | -CN |
| $C_3H_7$- | -COO- | $-C_6H_4-$ | $-C_6H_4-CN$ |
| $C_5H_{11}$- | -COO- | $-C_6H_4-$ | $-C_6H_4-CN$ |
| $C_3H_7$- | -COO- | $-C_6H_4-$ | $-C_6H_{10}-C_3H_7$ |
| $C_4H_9$- | -COO- | $-C_6H_4-$ | $-C_6H_{10}-C_3H_7$ |
| $C_5H_{11}$- | -COO- | $-C_6H_4-$ | $-C_6H_{10}-C_3H_7$ |
| $C_3H_7$- | -COO- | $-C_6H_4-$ | $-C_6H_{10}-C_4H_9$ |
| $C_4H_9$- | -COO- | $-C_6H_4-$ | $-C_6H_{10}-C_4H_9$ |
| $C_5H_{11}$- | -COO- | $-C_6H_4-$ | $-C_6H_{10}-C_4H_9$ |
| $C_3H_7$- | -COO- | $-C_6H_4-$ | $-C_6H_{10}-C_5H_{11}$ |
| $C_4H_9$- | -COO- | $-C_6H_4-$ | $-C_6H_{10}-C_5H_{11}$ |
| $C_5H_{11}$- | -COO- | $-C_6H_4-$ | $-C_6H_{10}-C_5H_{11}$ |
| $C_3H_7$- | -COO- | $-C_6H_{10}-$ | $-C_6H_4-C_3H_7$ |
| $C_5H_{11}$- | -COO- | $-C_6H_{10}-$ | $-C_6H_4-C_3H_7$ |
| $C_3H_7$- | -COO- | $-C_6H_{10}-$ | $-C_6H_4-C_5H_{11}$ |
| $C_5H_{11}$- | -COO- | $-C_6H_{10}-$ | $-C_6H_4-C_5H_{11}$ |
| $C_3H_7$- | -COO- | $-C_6H_4-$ | $-C_6H_4-C_5H_{11}$ |
| $C_3H_7$- | -COO- | $-C_6H_{10}-$ | $-C_6H_{10}-C_5H_{11}$ |

Tabelle 1 (Fortsetzung)

| $R^1$ | X | Ring A | $R^2$ |
|---|---|---|---|
| $C_3H_7$- | -OOC- | $-C_6H_{10}-$ | -CN |
| $C_5H_{11}$- | -OOC- | $-C_6H_{10}-$ | -CN |
| $C_3H_7$- | -OOC- | $-C_6H_{10}-$ | $-C_6H_4-CN$ |
| $C_5H_{11}$- | -OOC- | $-C_6H_{10}-$ | $-C_6H_4-CN$ |
| $C_3H_7$- | -OOC- | $-C_6H_{10}-$ | $-C_6H_4-C_3H_7$ |
| $C_4H_9$- | -OOC- | $-C_6H_{10}-$ | $-C_6H_4-C_3H_7$ |
| $C_5H_{11}$- | -OOC- | $-C_6H_{10}-$ | $-C_6H_4-C_3H_7$ |
| $C_3H_7$- | -OOC- | $-C_6H_{10}-$ | $-C_6H_4-C_5H_{11}$ |
| $C_5H_{11}$- | -OOC- | $-C_6H_{10}-$ | $-C_6H_4-C_5H_{11}$ |
| $C_3H_7$- | -OOC- | $-C_6H_{10}-$ | $-C_6H_{10}-C_5H_{11}$ |
| $C_5H_{11}$- | -OOC- | $-C_6H_{10}-$ | $-C_6H_{10}-C_3H_7$ |

Die Verbindungen der Formel I können erfindungsgemäss dadurch hergestellt werden, dass man

(a) zur Herstellung der Verbindungen der Formel I, worin X die Gruppe —COO— oder —OOCbedeutet, eine Verbindung der allgemeinen Formel

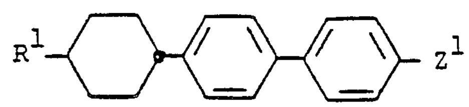

(II)

und eine Verbindung der allgemeinen Formel

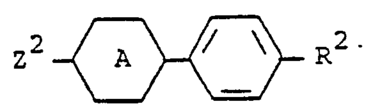

(III)

worin eine der Gruppen $Z^1$ und $Z^2$ die Carboxylgruppe und die andere die Hydroxygruppe bezeichnet und $R^1$, $R^2$ und Ring A die in Formel I gegebenen Bedeutungen haben,
oder reaktionsfähige Derivate dieser Verbindungen verestert, oder

b) zur Herstellung der Verbindungen der Formel I, worin X die Gruppe $—CH_2CH_2—$ bedeutet, eine Verbindung der allgemeinen Formel

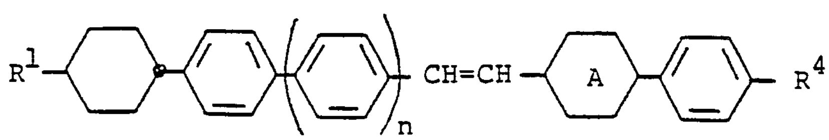

(IV)

worin $R^4$ Cyano oder p-Cyanophenyl darstellt und $R^1$, n und Ring A die in Formel I gegebenen Bedeutungen haben,
katalytisch hydriert.

Die Veresterung der Verbindungen der Formeln II und III kann in an sich bekannter Weise durch Veresterung der Carbonsäure oder eines reaktionsfähigen Derivates (z. B. Säurechlorid, -bromid oder -anhydrid) mit der Hydroxyverbindung oder eines geeigneten Salzes (z. B. des Natriumsalzes) erfolgen. Eine bevorzugte Methode ist die Umsetzung des Säurechlorids (welches aus der Carbonsäure z. B. durch Erhitzen mit Thionylchlorid erhaltlich ist) mit der Hydroxyverbindung. Diese Umsetzung wird zweckmässig in einem inerten organischen Lösungsmittel, beispielsweise einem Aether (wie Diäthyläther oder Tetrahydrofuran) oder Dimethylformamid, Benzol, Toluol, Cyclohexan, Tetrachlorkohlenstoff und dergleichen durchgeführt. Der frei werdende Chlorwasserstoff kann mit einem Säurebindemittel (z. B. einem tertiären Amin oder Pyridin) gebunden werden. Das Säurebindemittel kann auch zugleich als Lösungsmittel dienen. Weitere bevorzugte Methoden sind die Umsetzung der Carbonsäure mit der Hydroxyverbindung in Gegenwart von 4-(Dimethylamino) pyridin und Dicyclohexylcarbodiimid oder in Gegenwart von Oxalylchlorid und Dimethylformamid. Temperatur und Druck der obigen Veresterungsreaktion sind nicht kritisch. Im allgemeinen werden jedoch Atmosphärendruck und eine Temperatur zwischen etwa —30 °C und der Siedetemperatur des Reaktions-gemisches angewendet.

Die katalytische Hydrierung der Verbindungen der Formel IV kann in an sich bekannter Weise und mit gebräuchlichen Hydrierkatalysatoren erfolgen, beispielsweise mit Palladium, Platin, Raney-Nickel und dergleichen, vorzugsweise mit Palladium. Als Lösungsmittel können irgendwelche inerte organische Lösungsmittel, wie gesättigte Alkohole, Aether, Ester, Carbonsäuren, Kohlenwasserstoffe und dergleichen, beispielsweise Aethanol, Dioxan, Tetrahydrofuran, Essigsäureäthylester, Eisessig, Toluol oder Hexan, verwendet werden. Temperatur und Druck sind nicht kritisch. Im allgemeinen werden jedoch eine Temperatur zwischen Raumtemperatur und der Siedetemperatur des Reaktionsgemisches und ein Druck von etwa 1 bis etwa 5 Atmosphären angewendet.

Die Verbindungen der Formel IV sind neu und ebenfalls Gegenstand der vorliegenden Erfindung. Die trans-Isomere der Verbindungen der Formel IV (d. h. die Verbindungen mit trans-ständiger Doppelbindung) sind flüssigkristalline Verbindungen mit sehr grossen Mesophasenbereichen.

Die Verbindungen der Formel IV können nach den an sich bekannten Methoden der Wittig-Reaktion aus [(4'-(trans-4-Alkylcyclohexyl)-4-biphenylyl) methyl] triphenyl-phosphoniumbromid bzw. [p-(trans-4-Alkylcyclohexyl) benzyl]-triphenylphosphoniumbromid und einem Aldehyd der allgemeinen Formel

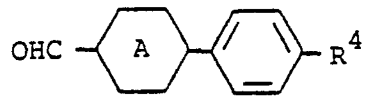

worin Ring A p-Phenylen oder trans-1,4-Cyclohexylen bezeichnet und $R^4$ Cyano oder p-Cyanophenyl darstellt,
erhalten werden.

Die Aldehyde der Formel V sind neu und ebenfalls Gegenstand der Erfindung.

Die Verbindungen der Formel V, worin Ring A p-Phenylen bezeichnet, können beispielsweise dadurch erhalten werden, dass man Biphenyl-4,4'-dicarbonsäure bzw. p-Terphenyl-4,4"-dicarbonsäure zum Monoester umsetzt (z. B. mit Diazomethan), diesen mit Thionylchlorid, Ammoniak und Phosphoroxychlorid über das Säurechlorid und Amid in den Cyanoester überführt, den Cyanoester mit Natronlauge verseift und die Cyanosäure durch Umsetzung mit Thionylchlorid und Rosenmund-Reduktion in den Cyanoaldehyd überführt.

Die Verbindungen der Formel V, worin Ring A trans-1,4-Cyclohexylen bezeichnet, können beispielsweise durch Wittig-Reaktion von 4-(p-Cyanophenyl) cyclohexanon bzw. 4-(4'-Cyano-4-biphenylyl) cyclohexanon mit Triphenylmethoxymethylphosphoniumchlorid und anschliessende Hydrolyse des Enoläthers hergestellt werden.

Die Verbindungen der Formel III, worin $Z^2$ die Carboxylgruppe bezeichnet und $R^2$ Cyano oder p-Cyanophenyl bedeutet, können beispielsweise aus den Verbindungen der Formel V durch Jones-Oxidation oder durch Oxidation mit Pyridiniumdichromat hergestellt werden. Die Verbindungen der Formel III, worin $Z^2$ die Hydroxygruppe bezeichnet und $R^2$ Cyano oder p-Cyanophenyl bedeutet, können beispielsweise durch Reduktion von 4-(p-Cyanophenyl) cyclohexanon bzw. 4-(4'-Cyano-4-biphenylyl) cyclohexanon mit Natriumborhydrid erhalten werden. Die übrigen Verbindungen der Formeln II und III sind bekannte oder Analoge bekannter Verbindungen und können nach bekannten Methoden hergestellt werden.

Die Verwendung der erfindungsgemässen Verbindungen für oder als flüssigkristalline stationäre Phasen in der Gaschromatographie kann in an sich bekannter Weise und auf üblichen Trägermaterialien erfolgen. Die erfindungsgemässen Verbindungen können in reiner Form oder auch als Mischungen von 2 oder mehreren Verbindungen der Formel I untereinander oder als Mischungen von einer oder mehreren Verbindungen der Formel I mit einer oder mehreren weiteren geeigneten Substanzen eingesetzt werden.

Die Erfindung betrifft daher auch flüssigkristalline Gemische mit mindestens 2 Komponenten, welche dadurch gekennzeichnet sind, dass mindestens eine Komponente eine Verbindung der obigen Formel I ist.

Um eine allmähliche Aenderung der Zusammensetzung bei längerem Gebrauch zu vermeiden, werden für chromatographische Anwendungen vorzugsweise nur Verbindungen mit vergleichbar niedrigen Dampfdrücken gemischt. Der Anteil an Verbindungen der Formel I in den flüssigkristallinen Gemischen für chromatographische Zwecke kann in breiten Grenzen variieren und etwa 1-100 Gew.-% betragen. Im allgemeinen beträgt der Anteil an Verbindungen der Formel I jedoch mindestens etwa 50 Gew.-%.

Die erfindungsgemässen Verbindungen eignen sich ebenfalls für elektro-optische Anwendungen und können in Form von Gemischen mit anderen, für flüssigkristalline Dielektrika geeigneten Substanzen verwendet werden, wie z. B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Terphenyle, Phenylcyclohexane, Cyclohexylbiphenyle, Phenylpyrimidine, Phenyldioxane, 2-Cyclohexyl-1-phenyläthane, Pyridazine, 2,3-Dicyano-1,4-phenylen-Derivate und dergleichen. Derartige Substanzen sind dem Fachmann bekannt und viele davon sind zudem im Handel erhältlich. Der Anteil an Verbindungen der Formel I in flüssigkristallinen Mischungen für elektro-optische Anwendungen beträgt bevorzugt etwa 1-30 Gew.-% und besonders bevorzugt etwa 2-15 Gew.-%.

Wegen der stark klärpunktserhöhenden Wirkung der erfindungsgemässen Verbindungen eignen sich diese vor allem für flüssigkristalline Dielektrika, welche Substanzen mit relativ kleinen Mesophasenbereichen oder sogar monotrope oder nicht-flüssigkristalline Substanzen enthalten. Bevorzugt sind insbesondere diejenigen Gemische, welche eine oder mehrere Verbindungen der Formel I und eine oder mehrere der Verbindungen der folgenden allgemeinen Formeln

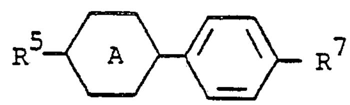
(VI)

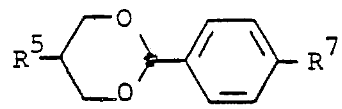
(VII)

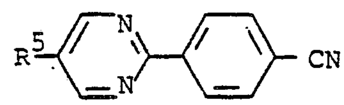
(VIII)

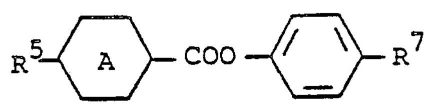
(IX)

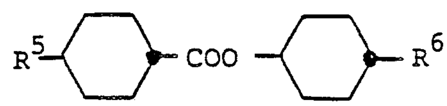
(X)

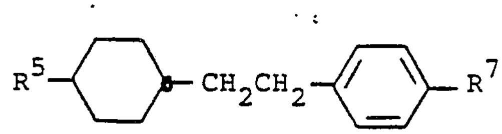
(XI)

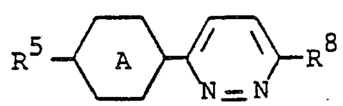
(XII)

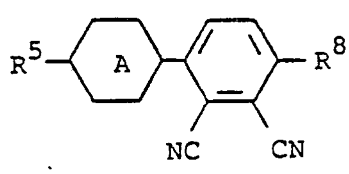
(XIII)

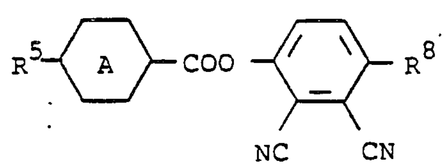
(XIV)

worin Ring A für p-Phenylen oder trans-1,4-Cyclohexylen steht, $R^5$ und $R^6$ geradkettiges $C_1$-$C_7$-Alkyl darstellen, $R^7$ Cyano, geradkettiges $C_1$-$C_7$-Alkyl oder geradkettiges $C_1$-$C_7$-Alkoxy bezeichnet und $R^8$ geradkettiges $C_1$-$C_7$-Alkyl oder geradkettiges $C_1$-$C_7$-Alkoxy bedeutet,
enthalten.

Die erfindungsgemässen Dielektrika können ferner geeignete, optisch aktive Verbindungen (z. B.

optisch aktive Biphenyle) und/oder dichroitische Farbstoffe (z. B. Azo-, Azoxy- oder Anthrachinon-Farbstoffe) enthalten. Der Anteil solcher Verbindungen wird durch die Löslichkeit und die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-%.

Die Herstellung der flüssigkristallinen Mischungen und der elektro-optischen Vorrichtungen kann in an sich bekannter Weise erfolgen.

Die Herstellung der erfindungsgemässen Verbindungen und deren Verwendung in der Chromatographie wird anhand der nachstehenden Beispiele weiter veranschaulicht. C bezeichnet eine kristalline, S eine smektische, N eine nematische und I die isotrope Phase.

Beispiel 1

2,029 g p-[trans-4-[2-(4'-(trans-4-Pentylcyclohexyl)-4-biphenylyl) vinyl] cyclohexyl] benzonitril wurden in 150 ml Toluol/Aethanol (Vol. 4 : 1) gelöst, mit 0,3 g Palladium/Kohle (10 %) versetzt und bei Raumtemperatur hydriert, bis die Wasserstoffaufnahme zum Stillstand kam. Anschliessend wurde das Reaktionsgemisch genutscht, der Rückstand mit Dichlormethan nachgewaschen und das Filtrat eingedampft. Das erhaltene, farblose, feste Rohprodukt wurde in Toluol gelöst und an Kieselgel mit Toluol chromatographiert. Hierbei wurden 1,255 g Substanz isoliert, welche aus Dichlormethan/Hexan umkristallisiert und im Hochvakuum bei 20 °C über Phosphorpentoxid getrocknet wurde. Ausbeute : 0,894 g reines p-[trans-4-[2-(4'-(trans-4-Pentylcyclohexyl)-4-biphenylyl) äthyl] cyclohexyl] benzonitril : (C-C) 134,5-135 °C, Smp. (C-S) 147-147,5 °C, (S-N) 167,5-169 °C, Klp. (N-I) 370 °C.

Das als Ausgangsmaterial verwendete p-[trans-4-[2-(4'-(trans-4-Pentylcyclohexyl)-4-biphenylyl) vinyl] cyclohexyl] benzonitril wurde wie folgt hergestellt :

a) Eine Suspension von 10,4 g Triphenyl-methoxymethylphosphoniumchlorid in 60 ml t-Butylmethyläther wurde bei — 10 °C unter Argonbegasung vorgelegt und innert 10 Minuten mit 3,6 g festem Kalium-t-butylat versetzt. Nach beendeter Zugabe wurde das Reaktionsgemisch noch 30 Minuten bei — 10 °C bis 0 °C gerührt und dann bei 0 °C tropfenweise mit einer Lösung von 4,2 g 4-(p-Cyanophenyl) cyclohexanon in 50 ml absolutem Tetrahydrofuran versetzt. Anschliessend wurde das Reaktionsgemisch noch 2 Stunden bei Raumtemperatur gerührt, dann auf 500 ml Hexan gegossen und filtriert. Niederdruckchromatographie (0,5 bar) des eingeengten Rückstandes (7,1 g) an Kieselgel mit Essigester/Petroläther (Vol. 1 : 19) ergab 4,5 g (94 %) p-[4-(Methoxymethylen) cyclohexyl] benzonitril als farbloses, allmählich kristallisierendes Oel (Reinheit 95 %) ; Rf-Wert (Essigester/Petroläther Vol. 1 : 9) = 0,30.

b) Ein Gemisch von 14,25 g p-[4-(Methoxymethylen) cyclohexyl] benzonitril (Reinheit 96,1 %) und 200 ml Tetrahydrofuran/2N Salzsäure (Vol. 4 : 1) wurde 30 Minuten zum Rückfluss erhitzt. Anschliessend wurde das Reaktionsgemisch auf 300 ml Wasser gegossen und dreimal mit je 200 ml Diäthyläther extrahiert. Die organischen Phasen wurden mit 200 ml Wasser gewaschen, über Magnesiumsulfat getrocknet und eingeengt. Hierbei wurden 13,75 g (103 %) Rohprodukt von 4-(p-Cyanophenyl) cyclohexancarboxaldehyd (trans/cis-Verhältnis ca. 3 : 1) als farbloses, langsam kristallisierendes Oel erhalten. Umkristallisation dieses Materials aus 1,3 l Hexan ergab 3,71 g (29 %) trans-4-(p-Cyanophenyl) cyclohexancarboxaldehyd (Reinheit 99,5 %) als lange, farblose Nadeln mit Smp. 57,1 °C. Die Mutterlauge wurde auf ein Volumen von 1 l eingeengt und nochmals zur Kristallisation angesetzt, wobei als 2. Kristallisat 1,20 g farblose Nadeln enthaltend 97 % trans-Aldehyd und 3 % cis-Aldehyd erhalten wurden. Die anfallende Mutterlauge wurde nun eingeengt, der Rückstand nochmals in 100 ml Tetrahydrofuran/2N Salzsäure (Vol. 4 : 1) 30 Minuten zum Rückfluss erhitzt und dann das Gemisch wie beim ersten Mal aufgearbeitet. Das erhaltene Rohprodukt wurde mit dem 2. Kristallisat (1,2 g) vereinigt und aus 900 ml Hexan umkristallisiert, wobei nochmals 3,56 g (28 %) trans-4-(p-Cyanophenyl) cyclohexancarboxaldehyd (Reinheit 99,7 %) als lange, farblose Nadeln isoliert werden konnten. Die Mutterlauge (enthaltend 4,2 g Rohprodukt) wurde nicht mehr aufgearbeitet. Gesamtausbeute nach 2 Zyklen : 7,27 g (57 %) trans-4-(p-Cyanophenyl) cyclohexancarboxaldehyd ; Smp. 57,1 °C.

c) 4,652 g [(4'-(trans-4-Pentylcyclohexyl)-4-biphenylyl)-methyl]-triphenylphosphoniumbromid wurden unter Rühren und Stickstoffbegasung in 40 ml absolutem t-Butylmethyläther suspendiert, dann das Gemisch mit 0,789 g Kalium-t-butylat versetzt und noch 1 Stunde bei Raumtemperatur gerührt. Anschliessend wurde die orange Suspension auf — 60 °C abgekühlt und innert 15 Minuten tropfenweise mit einer Lösung von 1,0 g trans-4-(p-Cyanophenyl) cyclohexancarboxaldehyd in 8 ml absolutem t-Butylmethyläther versetzt. Durch den Tropftrichter wurden 5 ml absoluter t-Butylmethyläther nachgespült und dann die Temperatur des Reaktionsgemisches innert 1,25 Stunden auf — 25 °C ansteigen gelassen. Anschliessend wurde das Reaktionsgemisch auf 75 ml Wasser gegossen und dreimal mit Toluol extrahiert. Die organischen Extrakte wurden einmal mit Wasser gewaschen, über Natriumsulfat getrocknet und im Vakuum eingedampft. Das zurückbleibende, feste, farblose Produkt (5,0 g) wurde in Hexan suspendiert und an Kieselgel chromatographiert. Hexan und Hexan/Toluol (Vol. 9 : 1) eluierten 0,653 g farbloses 4-Methyl-4'-(trans-4-pentylcyclohexyl) biphenyl [Smp. (C-N) 98,9 °C, Klp. (N-I) 175,3 °C] und Hexan/Toluol

(Vol. 1 : 1), Toluol und Toluol/Aceton (enthaltend 1-10 Vol-% Aceton) eluierten schliesslich 2,029 g p-[trans-4-[2-(4'-(trans-4-Pentylcyclohexyl)-4-biphenylyl)-vinyl] cyclohexyl] benzonitril als farbloses bis gelbliches Reaktionsprodukt, welches ohne weitere Reinigung für die anschliessende Hydrierung verwendet wurde.

In analoger Weise wurden folgende Verbindungen hergestellt :

p-[trans-4-[2-(4-(trans-4-Pentylcyclohexyl) phenyl)-äthyl] cyclohexyl] benzonitril ; Smp. (C-N) 99,6 °C, Klp. (N-I) 265,0 °C, (S-N) 76,5 °C,

p-[trans-4-[2-(4'-(trans-4-Butylcyclohexyl)-4-biphenylyl) äthyl] cyclohexyl] benzonitril ; Smp. (C-N) 174,6 °C, Klp. (N-I) 378,5 °C.

Beispiel 2

Ein Gemisch von 1,0 g trans-4-(p-Cyanophenyl) cyclohexanol, 1,75 g 4'-(trans-4-Pentylcyclohexyl)-4-biphenylcarbonsäure, 1,2 g Dicyclohexylcarbodiimid und 98 mg 4-(Dimethylamino) pyridin in 60 ml Dichlormethan wurde bei Raumtemperatur unter Argonbegasung 18 Stunden gerührt. Anschliessend wurde die beige Suspension filtriert (Nachwaschen mit Dichlormethan) und das Filtrat eingeengt. Niederdruckchromatographie (0,5 bar) des Rückstandes (3,1 g) an Kieselgel mit Toluol lieferte 1,2 g (45 %) reinen 4'-(trans-4-Pentylcyclohexyl)-4-biphenylcarbonsäure-trans-4-(p-cyanophenyl) cyclohexylester. Umkristallisation aus Essigester/Aceton (Vol. 1 : 4) ergab weisse Kristalle mit Smp. (C-N) 148,6 °C und Klp. (N-I) 347 °C ; Reinheit 99,6 %.

Das als Ausgangsmaterial verwendete trans-4-(p-Cyanophenyl) cyclohexanol wurde wie folgt hergestellt :

51,0 g 4-(p-Cyanophenyl) cyclohexanon wurden in 1,5 l Aethanol/Wasser (Vol. 4 : 1) fast völlig gelöst und dann innert 10 Minuten mit 9,68 g Natriumborhydrid versetzt. Das Gemisch wurde noch 30 Minuten gerührt und dann über Nacht stehen gelassen. Anschliessend wurde das Reaktionsgemisch vorsichtig mit 350 ml verdünnter, eiskalter Salzsäure (1 Teil konzentrierte Salzsäure auf 4 Teile Wasser) versetzt, dann mit 2 l Wasser und 1 l Diäthyläther versetzt und geschüttelt. Die wässrige Phase wurde abgetrennt und viermal mit je 0,5 l Diäthyläther extrahiert. Die vereinigten organischen Phasen wurden mit 100 ml gesättigter Natriumhydrogencarbonat-Lösung und mit 100 ml gesättigter Kochsalzlösung gewaschen, über Natriumsulfat getrocknet und eingeengt. Das noch wasserhaltige Rohprodukt wurde durch Lösen in Toluol und Aceton und Einengen weiter getrocknet. Der beige, feste Rückstand (48,9 g) wurde in 300 ml Toluol heiss gelöst und von einem feinen, ungelösten Rückstand heiss abfiltriert. Das Filtrat wurde auf — 20 °C gekühlt und das erhaltene Kristallisat abgenutscht, mit 50 ml Toluol/Hexan (Vol. 9 : 1) gewaschen und getrocknet. Hierbei wurden 31,72 g (61,6 %) reines, hellbeiges trans-4-(p-Cyanophenyl)-cyclohexanol mit Smp. 119,5-122,4 °C erhalten. Aus der Mutterlauge wurden durch Kristallisation aus 60 ml Toluol bei — 20 °C weitere 3,21 g (6,2 %) trans-4-(p-Cyanophenyl)-cyclohexanol mit Smp. 116,5-122,1 °C gewonnen.

In analoger Weise wurden folgende Verbindungen hergestellt :

4'-(trans-4-Propylcyclohexyl)-4-biphenylcarbonsäure-trans-4-(p-cyanophenyl) cyclohexylester ; Smp. (C-N) 189,6 °C, Klp. (N-I) 350 °C,

4'-(trans-4-Butylcyclohexyl)-4-biphenylcarbonsäure-trans-4-(p-cyanophenyl) cyclohexylester ; Smp. (C-N) 168,6 °C bzw. 169,8 °C (2 Modifikationen), Klp. (N-I) 348,5 °C.

Beispiel 3

Eine Lösung von 2,40 g 4-Hydroxy-4'-(trans-4-pentylcyclohexyl) biphenyl und 0,149 g 4-(Dimethylamino) pyridin in 125 ml absolutem Dichlormethan wurde unter Rühren mit 2,40 g 4'-(trans-4-Propylcyclohexyl)-4-biphenylcarbonsäure versetzt. Die gelbliche Suspension wurde mit 25 ml absolutem Dichlormethan verdünnt, mit 1,843 g festem Dicyclohexylcarbodiimid und nochmals mit 35 ml absolutem Dichlormethan (Nachspülen) versetzt, dann 24 Stunden bei Raumtemperatur gerührt und anschliessend 4 Stunden zum Rückfluss erhitzt. Nach Abkühlen auf Raumtemperatur wurde der Niederschlag genutscht, mit Dichlormethan gewaschen und getrocknet, wobei 3,057 g farbloses, festes Produkt erhalten wurden. Einengen des Filtrates im Vakuum bis zur Trockene ergab 3,7 g gelbe, feste Substanz, welche in Dichlormethan suspendiert und zusammen mit 15 g Kieselgel im Vakuum eingedampft wurde. Der Rückstand wurde in Toluol suspendiert und auf eine Säule von Kieselgel in Toluol gegeben. Toluol, Toluol/1 % Aceton und Toluol/2 % Aceton eluierten 1,823 g rohen Ester. Nach mehrmaliger Umkristallisation aus Dichlormethan/Hexan und Trocknung im Hochvakuum über Phosphorpentoxid während 16 Stunden wurden 0,762 g reiner 4'-(trans-4-Propylcyclohexyl)-4-biphenylcarbonsäure-4'-(trans-4-pentylcyclohexyl)-4-biphenylylester erhalten, (C-C) 132 °C, Smp. (C-S) 200 °C, (S-S) 219 °C, (S-N) 224 °C, Klp. (N-I) 462 °C. Das zuerst erhaltene, farblose, feste Produkt (3,057 g wurde zusammen mit den Mutterlaugen aus den obigen Umkristallisationen in Dichlormethan suspendiert und an Kieselgel in Dichlormethan chromatographiert. Dichlormethan und Dichlormethan/2 % Aceton eluierten 2,467 g gelblichen, kristallinen Ester. Mehrmalige Umkristallisation dieses Rohproduktes aus Dichlormethan/Hexan und Trocknung im Hochvakuum

über Phosphorpentoxid ergaben weitere 1,916 g reines Produkt und 0,502 g leicht verunreinigtes Produkt.

Beispiel 4

a) Eine Suspension von 1,07 g Aluminiumchlorid in 10 ml 1,2-Dichloräthan wurde bei Raumtemperatur unter Argonbegasung vorgelegt, innert 10 Minuten mit einer Lösung von 2,0 g 4-(4-Biphenylyl) cyclohexanon in 10 ml 1,2-Dichloräthan versetzt und noch 30 Minuten nachgerührt. Die entstandene braun-violette Suspension wurde bei Raumtemperatur innert 10 Minuten zu einem Gemisch von 1,07 g Aluminiumchlorid in 10 ml 1,2-Dichloräthan, welches vorgängig bei Raumtemperatur mit 1,37 ml Oxalylchlorid versetzt worden war, zugetropft. Das Reaktionsgemisch wurde noch 30 Minuten bei Raumtemperatur gerührt und dann unter Abkühlung auf ca. 0 °C innert 25 Minuten vorsichtig mit 30 ml einer 30 %-igen (Gew./Vol.) Kalziumchlorid-Lösung versetzt (stark exotherme Reaktion). Die entstandene, beige Suspension wurde auf 200 ml Eiswasser gegossen und dreimal mit je 200 ml Methylenchlorid extrahiert. Die organischen Phasen wurden zweimal mit je 100 ml Eiswasser gewaschen, über Magnesiumsulfat getrocknet und auf ein Volumen von ca. 35 ml eingeengt. Diese Lösung wurde in einem Sulfierkolben mit Tropftrichter und mechanischem Rührer innert 10 Minuten zu einem bei ca. 15 °C vorgelegten Gemisch von 4,5 ml 25 %-iger (Gew./Vol.) wässriger Ammoniak-Lösung und 3 ml Wasser zugegeben. Das heterogene Reaktionsgemisch wurde noch 1,75 Stunden bei Raumtemperatur gerührt. Dann wurde der entstandene, beige Niederschlag abfiltriert und mit Wasser gut gewaschen. Nach Trocknung am Hochvakuum wurden 1,66 g (71 %) 4'-(4-Oxocyclohexyl)-4-biphenylcarboxamid als beiges, amorphes Pulver erhalten ; Rf-Wert (Chloroform/Methanol Vol. 19 : 1) = 0,36.

b) Ein Gemisch von 1,50 g 4'-(4-Oxocyclohexyl)-4-biphenylcarboxamid und 20 ml Pyridin wurde unter Argonbegasung bei 0 °C vorgelegt und mit 463 µl Mesylchlorid versetzt. Das Kühlbad wurde entfernt und die gelbe Suspension 19 Stunden bei Raumtemperatur gerührt. Anschliessend wurden nochmals 1,4 ml Mesylchlorid bei Raumtemperatur zum Reaktionsgemisch zugegeben und das Gemisch bei 60 °C 67 Stunden gerührt. Das abgekühlte, dunkelbraune Reaktionsgemisch wurde mit 40 ml eiskalter, 25 %-iger (Gew./Vol.) Salzsäure sauer gestellt und dreimal mit je 100 ml Methylenchlorid extrahiert. Die organischen Phasen wurden dreimal mit je 50 ml Wasser gewaschen, über Magnesiumsulfat getrocknet und eingeengt. Niederdruckchromatographie (0,5 bar) des rotbraunen, kristallinen Rückstandes (0,9 g) an Kieselgel mit Chloroform ergab im Hauptlauf nach zusätzlicher Behandlung mit Aktivkohle in Chloroform bei 60 °C 300 mg (21 %) 4-(4'-Cyano-4-biphenylyl) cyclohexanon als leicht gelbe Kristalle ; Rf-Wert (Chloroform) = 0,43.

c) 4-(4'-Cyano-4-biphenylyl) cyclohexanon kann in analoger Weise zu Beispiel 1 in trans-4-(4'-Cyano-4-biphenylyl)-cyclohexancarboxaldehyd und 4'-[trans-4-[2-(4'-(trans-4-Alkylcyclohexyl)-4-biphenylyl) äthyl] cyclohexyl]-4-biphenylcarbonitril übergeführt werden. In analoger Weise zu Beispiel 2 kann ferner 4-(4'-Cyano-4-biphenylyl) cyclohexanon zu trans-4-(4'-Cyanobiphenylyl) cyclohexanol und 4'-(trans-4-Alkylcyclohexyl)-4-biphenylcarbonsäure-trans-4-(4'-cyanobiphenylyl) cyclohexylester weiter umgesetzt werden.

Beispiel 5

Eine Lösung von 0,214 g 4'-[trans-4-[2-(4'-(trans-4-Pentylcyclohexyl)-4-biphenylyl) vinyl] cyclohexyl]-4-biphenylcarbonitril in 200 ml Tetrahydrofuran wurde mit 52,5 mg Palladium/Kohle (5 %) versetzt und bei Raumtemperatur hydriert, bis die Wasserstoffaufnahme zum Stillstand kam (1,75 Stunden). Das Reaktionsgemisch wurde anschliessend mit Stickstoff begast und genutscht (Nachspülen mit heissem Tetrahydrofuran). Nach Einengen des Filtrats im Vakuum und Trocknen bei 50 °C unter Wasserstrahlvakuum wurden 0,310 g farbloses, festes 4'-[trans-4-[2-(4'-(trans-4-Pentylcyclohexyl)-4-biphenylyl) äthyl] cyclohexyl]-4-biphenylcarbonitril erhalten, welches noch Lösungsmittel enthielt. Umkristallisation aus Tetrahydrofuran ergab farblose Nadeln mit Smp. (C-N) 194,5 °C, Klp. (N-I) 440 °C.

Das als Ausgangsmaterial verwendete 4'-[trans-4-[2-(4'-(trans-4-Pentylcyclohexyl)-4-biphenylyl) vinyl] cyclohexyl]-4-biphenylcarbonitril wurde wie folgt hergestellt :

In einem Sulfierkolben mit Stickstoffbegasung wurde unter Rühren eine Suspension von 2,337 g [(4'-(trans-4-Pentylcyclohexyl)-4-biphenylyl) methyl] triphenylphosphoniumbromid in 35 ml trockenem Tetrahydrofuran mit 0,394 g Kalium-t-butylat versetzt (Nachspülen mit 20 ml absolutem Tetrahydrofuran) und das Gemisch noch 1 Stunde bei Raumtemperatur gerührt. Nach dem Abkühlen auf — 60 °C wurde das Gemisch innert 25 Minuten tropfenweise mit einer Lösung von 0,928 g trans-4-(4'-Cyano-4-biphenylyl) cyclohexancarboxaldehyd [hergestellt aus 4-(4'-Cyano-4-biphenylyl) cyclohexanon in analoger Weise zu Beispiel 1 a) und b)] in 50 ml absolutem Tetrahydrofuran versetzt (Nachspülen mit 10 ml absolutem Tetrahydrofuran), dann die Suspension innert 3 Stunden auf 7 °C erwärmen gelassen und schliesslich über Nacht bei Raumtemperatur weitergerührt. Danach wurde die Suspension auf 100 ml Wasser gegossen. Der Niederschlag wurde abgenutscht, gut mit Wasser gewaschen und getrocknet, wobei 1,805 g festes, fast farbloses Produkt erhalten wurden. Aus dem Filtrat fiel ein feiner Niederschlag aus,

welcher nach Verdünnen mit Wasser auf 500 ml ebenfalls abgenutscht, mit Wasser gewaschen und getrocknet wurde (0,222 g gelbliche, feste Substanz). Die beiden Niederschläge (2,027 g) wurden mit 75 ml Diäthyläther während 10 Minuten bei Raumtemperatur verrührt und anschliessend genutscht, mit Diäthyläther gewaschen und getrocknet. Die erhaltene farblose Festsubstanz (1,074 g) wurde in 75 ml Methylenchlorid suspendiert und dann der Niederschlag abgenutscht, mit Methylenchlorid gewaschen und getrocknet. Hierbei wurden 0,871 g rohes 4'-[trans-4-[2-(4'-(trans-4-Pentylcyclohexyl)-4-biphenylyl)-vinyl] cyclohexyl]-4-biphenylcarbonitril als farblose Festsubstanz (Rf-Wert = 0,46 in Toluol) erhalten, welche ohne zusätzliche Reinigung weiter umgesetzt wurde.

Beispiel 6

Auf einer 2 m langen, gepackten Säule (innerer Durchmesser 2,2 mm), welche mit 2 Gew.-% 4'-(trans-4-Propylcyclohexyl)-4-biphenylcarbonsäure-4'-(trans-4-pentylcyclohexyl)-4-biphenylylester auf Gaschrom Q® (Applied Science Labs), 120/140 mesh, gefüllt war, wurde bei einer Analysentemperatur von 290 °C ein Isomerengemisch von p-[5-(cis/trans-4-Pentylcyclohexyl)-2-pyrimidinyl] benzonitril getrennt. Als Trägergas diente Stickstoff (30 ml/Min.). Die Retentionszeit betrug für das cis-Isomere 4,3 Minuten und für das trans-Isomere 16 Minuten. Die relative Retention α für das trans-Isomere betrug somit 3,7 (α = 1,0 für das cis-Isomere).

Beispiel 7

Auf einer 2 m langen, gepackten Säule (innerer Durchmesser 2,2 mm), welche mit 2 Gew.-% 4'-(trans-4-Pentylcyclohexyl)-4-biphenylcarbonsäure-trans-4-(p-cyanophenyl-cyclohexylester auf Gaschrom Q® (Applied Science Labs), 120/140 mesh, gefüllt war, wurde bei einer Analysentemperatur von 150 °C ein Gemisch von Oleinsäuremethylester [(Z)-Isomer] und Elaidinsäuremethylester [(E)-Isomer] getrennt. Als Trägergas diente Stickstoff (30 ml/Min.). Die Retentionszeit betrug für den Oleinsäuremethylester 18,5 Minuten und für den Elaidinsäuremethylester 20,8 Minuten. Die relative Retention α für Elaidinsäuremethylester betrug somit 1,13 (α = 1,0 für Oleinsäuremethylester).

Beispiel 8

Auf einer 2 m langen, gepackten Säule (innerer Durchmesser 2,2 mm), welche mit 2 Gew.-% p-[trans-4-[2-(4'-(trans-4-Pentylcyclohexyl)-4-biphenylyl) äthyl] cyclohexyl]-benzonitril auf Gaschrom Q® (Applied Science Labs), 120/140 mesh, gefüllt war, wurde bei einer Analysentemperatur von 180 °C ein Gemisch von α-Naphthol und β-Naphthol getrennt. Als Trägergas diente Stickstoff (30 ml/Min.). Die Retentionszeit betrug für α-Naphthol 5,2 Minuten und für β-Naphthol 6,6 Minuten. Die relative Retention α für β-Naphthol betrug somit 1,2 (α = 1,0 für α-Naphthol).

Beispiel 9

Auf einer 2 m langen, gepackten Säule (innerer Durchmesser 2,2 mm), welche mit 2 Gew.-% 4'-[trans-4-[2-(4'-(trans-4-Pentylcyclohexyl)-4-biphenylyl) äthyl] cyclohexyl]-4-biphenylcarbonitril auf Gaschrom Q® (Applied Science Labs), 120/140 mesh, gefüllt war, wurde bei einer analysentemperatur von 230-300 °C und einer Aufheizrate von 4 °C/Minute ein Gemisch von Phenanthren (1), Anthracen (2), Fluoranthen (3), Pyren (4), Benz(a) anthracen (5), Chrysen (6), Benz(b) fluoranthen (7), Benz(k) fluoranthen (8) und Benz(a) pyren (9) getrennt. Als Trägergas diente Stickstoff (35 ml/Min.). Das Chromatogramm ist in Figur 1 dargestellt. Die Retentionszeit für Benz(a) pyren betrug 28 Minuten.

Beispiel 10

Auf einer 2 m langen, gepackten Säule (innerer Durchmesser 2,2 mm), welche mit 2 Gew.-% p-[trans-4-[2-(4-(trans-4-Pentylcyclohexyl) phenyl) äthyl] cyclohexyl]-benzonitril auf Gaschrom Q® (Applied Science Labs), 120/140 mesh, gefüllt war, wurde bei einer Analysentemperatur von 170 °C ein Gemisch enthaltend Oleinsäuremethylester [(Z)-Isomer] und Elaidinsäuremethylester [(E)-Isomer] getrennt. (Neben den beiden isomeren Estern enthielt das Gemisch noch Stearinsäuremethylester). Als Trägergas diente Stickstoff (35 ml/Min.). Die Retentionszeit betrug für Oleinsäuremethylester 13,5 Minuten und für Elaidinsäuremethylester 15 Minuten. Die relative Retention α für Elaidinsäuremethylester betrug somit 1,11 (α = 1,0 für Oleinsäuremethylester).

Beispiel 11

Auf einer 2 m langen, gepackten Säule (innerer Durchmesser 2,2 mm), welche mit 2 Gew.-% p-[trans-4-[2-(4-trans-4-Pentylcyclohexyl) phenyl) äthyl] cyclohexyl] benzonitril auf Gaschrom Q® (Applied Science Labs), 120/140 mesh, gefüllt war, wurde bei einer Analysentemperatur von 160 °C ein Isomerengemisch von 1-(p-Tolyl)-cis/trans-4-propylcyclohexan getrennt. Als Trägergas diente Stickstoff

(35 ml/Min.). Die Retentionszeit betrug für das cis Isomere 6 Minuten und für das trans-Isomere 13,5 Minuten. Die relative Retention α für 1-(p-Tolyl)-trans-4-propylcyclohexan betrug somit 2,4 [α = 1,0 für 1-(p-Tolyl)-cis-4-propyl-cyclohexan].

**Patentansprüche** (für die Vertragsstaaten : BE, CH, DE, FR, GB, IT, LI, NL)

1. Verbindungen der allgemeinen Formel

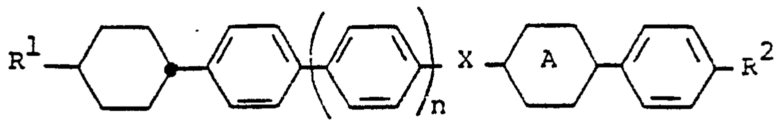

(I)

worin n für die Zahl 1 und X für die Gruppe —COO—, —OOC—, oder —$CH_2CH_2$— steht, oder n für die Zahl 0 und X für die Gruppe —$CH_2CH_2$— steht ; Ring A p-Phenylen oder trans-1,4-Cyclohexylen bezeichnet ; $R^2$ Cyano, p-Cyanophenyl oder, sofern X für —COO— oder —OOC— steht, auch p-$R^3$-Phenyl oder trans-4-$R^3$-Cyclohexyl darstellt ; und $R^1$ und $R^3$ geradkettiges $C_1$-$C_{10}$-Alkyl bedeuten.

2. Verbindungen nach Anspruch 1, dadurch gekennzeichnet, dass n für die Zahl 1 steht.

3. Verbindungen nach Anspruch 1 oder 2, dadurch gekennzeichnet, dass Ring A trans-1,4-Cyclohexylen und $R^2$ Cyano, p-Cyanophenyl oder p-$R^3$-Phenyl bezeichnen oder Ring A p-Phenylen und $R^2$ trans-4-$R^3$-Cyclohexyl bezeichnen.

4. Verbindungen nach einem der Ansprüche 1 bis 3, dadurch gekennzeichnet, dass X für die Gruppe —COO— steht.

5. Verbindungen nach einem der Ansprüche 1 bis 3, dadurch gekennzeichnet, dass X für die Gruppe —$CH_2CH_2$— steht.

6. Verbindungen nach einem der Ansprüche 1 bis 4, dadurch gekennzeichnet, dass $R^2$ p-$R^3$-Phenyl oder trans-4-$R^3$-Cyclohexyl darstellt und $R^3$ Propyl, Butyl oder Pentyl bedeutet.

7. Flüssigkristallines Gemisch mit mindestens 2 Komponenten, dadurch gekennzeichnet, dass mindestens eine Komponente eine Verbindung der in Anspruch 1 definierten Formel I ist.

8. Verfahren zur Herstellung der Verbindungen der in Anspruch 1 definierten Formel I, dadurch gekennzeichnet, dass man

a) zur Herstellung der Verbindungen der Formel I, worin X die Gruppe —COO— oder —OOCbedeutet, eine Verbindung der allgemeinen Formel

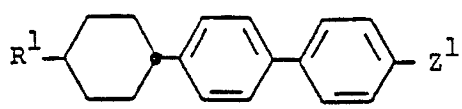

(II)

und eine Verbindung der allgemeinen Formel

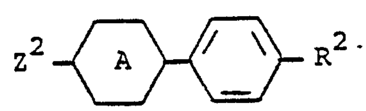

(III)

worin eine der Gruppen $Z^1$ und $Z^2$ die Carboxylgruppe und die andere die Hydroxygruppe bezeichnet und $R^1$, $R^2$ und Ring A die in Anspruch 1 gegebenen Bedeutungen haben,
oder reaktionsfähige Derivate dieser Verbindungen verestert, oder

b) zur Herstellung der Verbindungen der Formel I, worin X die Gruppe —$CH_2CH_2$— bedeutet, eine Verbindung der allgemeinen Formel

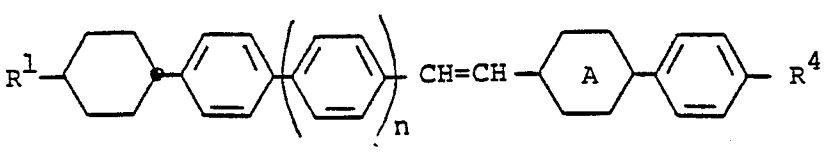

(IV)

worin $R^4$ Cyano oder p-Cyanophenyl darstellt und $R^1$, n und Ring A die in Anspruch 1 gegebenen Bedeutungen haben,
katalytisch hydriert.

9. Verwendung der Verbindungen der in Anspruch 1 definierten Formel I für elektro-optische Zwecke.

10. Verwendung der Verbindungen der in Anspruch 1 definierten Formel I in der Gaschromatographie.

**Patentansprüche** (für den Vertragsstaat AT)

1. Verfahren zur Herstellung der Verbindungen der allgemeinen Formel

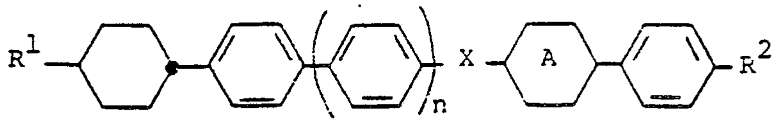

(I)

worin n für die Zahl 1 und X für die Gruppe —COO—, —OOC— oder —$CH_2CH_2$— steht, oder n für die Zahl 0 und X für die Gruppe —$CH_2CH_2$— steht ; Ring A p-Phenylen oder trans-1,4-Cyclohexylen bezeichnet ; $R^2$ Cyano, p-Cyanophenyl oder, sofern X für —COO— oder —OOC— steht, auch p-$R^3$-Phenyl oder trans-4-$R^3$-Cyclohexyl darstellt ; und $R^1$ und $R^3$ geradkettiges $C_1$-$C_{10}$-Alkyl bedeuten, dadurch gekennzeichnet, dass man

a) zur Herstellung der Verbindungen der Formel I, worin X die Gruppe —COO— oder —OOCbedeutet, eine Verbindung der allgemeinen Formel

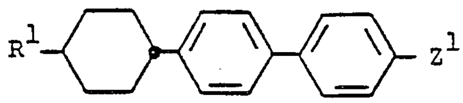

(II)

und eine Verbindung der allgemeinen Formel

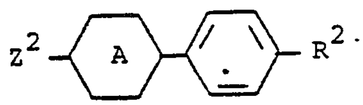

(III)

worin eine der Gruppen $Z^1$ und $Z^2$ die Carboxylgruppe und die andere die Hydroxygruppe bezeichnet und $R^1$, $R^2$ und Ring A die obigen Bedeutungen haben, oder reaktionsfähige Derivate dieser Verbindungen verestert, oder

b) zur Herstellung der Verbindungen der Formel I, worin X die Gruppe —$CH_2CH_2$— bedeutet, eine Verbindung der allgemeinen Formel

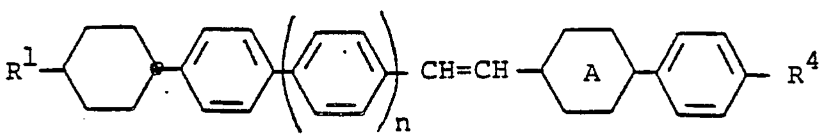

(IV)

worin $R^4$ Cyano oder p-Cyanophenyl darstellt und $R^1$, n und Ring A die obigen Bedeutungen haben, katalytisch hydriert.

2. Verfahren nach Anspruch 1, dadurch gekennzeichnet, dass n für die Zahl 1 steht.

3. Verfahren nach Anspruch 1 oder 2, dadurch gekennzeichnet, dass Ring A trans-1,4-Cyclohexylen und $R^2$ Cyano, p-Cyanophenyl oder p-$R^3$-Phenyl bezeichnen oder Ring A p-Phenylen und $R^2$ trans-4-$R^3$-Cyclohexyl bezeichnen.

4. Verfahren nach einem der Ansprüche 1 bis 3, dadurch gekennzeichnet, dass X für die Gruppe —COO— steht.

5. Verfahren nach einem der Ansprüche 1 bis 3, dadurch gekennzeichnet, dass X für die Gruppe —$CH_2CH_2$— steht.

6. Verfahren nach einem der Ansprüche 1 bis 4, dadurch gekennzeichnet, dass $R^2$ p-$R^3$-Phenyl oder trans-4-$R^3$-Cyclohexyl darstellt und $R^3$ Propyl, Butyl oder Pentyl bedeutet.

7. Flüssigkristallines Gemisch mit mindestens 2 Komponenten, dadurch gekennzeichnet, dass mindestens eine Komponente eine Verbindung der in Anspruch 1 definierten Formel I ist.

8. Verwendung der Verbindungen der in Anspruch 1 definierten Formel I für elektro-optische Zwecke.

9. Verwendung der Verbindungen der in Anspruch 1 definierten Formel I in der Gaschromatographie.

**Claims** (for the Contracting States : BE, CH, DE, FR, GB, IT, LI, NL)

1. Compounds of the general formula

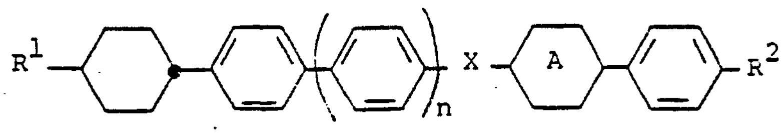

(I)

wherein n stands for the number 1 and X stands for the group —COO—, —OOC— or —$CH_2CH_2$—, or n stands for the number 0 and X stands for the group —$CH_2CH_2$— ; ring A denotes p-phenylene or trans-1,4-cyclohexylene ; $R^2$ represents cyano, p-cyanophenyl or, insofar as X stands for —COO— or —OOC—, also p-$R^3$-phenyl or trans-4-$R^3$-cyclohexyl ; and $R^1$ and $R^3$ signify straight-chain $C_1$-$C_{10}$-alkyl.

2. Compounds according to claim 1, characterized in that n stands for the number 1.

3. Compounds according to claim 1 or 2, characterized in that ring A denotes trans-1,4-cyclohexylene and $R^2$ denotes cyano, p-cyanophenyl or p-$R^3$-phenyl or ring A denotes p-phenylene and $R^2$ denotes trans-4-$R^3$-cyclohexyl.

4. Compounds according to any one of claims 1 to 3, characterized in that X stands for the group —COO—.

5. Compounds according to any one of claims 1 to 3, characterized in that X stands for the group —$CH_2CH_2$—.

6. Compounds according to any one of claims 1 to 4, characterized in that $R^2$ represents p-$R^3$-phenyl or trans-4-$R^3$-cyclohexyl and $R^3$ signifies propyl, butyl or pentyl.

7. A liquid crystalline mixture with least 2 components, characterized in that at least one component is a compound of formula I defined in claim 1.

8. A process for the manufacture of the compounds of formula I defined in claim 1, characterized by

a) for the manufacture of the compounds of formula I in which X signifies the group —COO— or —OOC—, esterifying a compound of the general formula

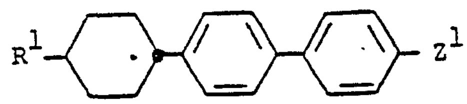

(II)

and a compound of the general formula

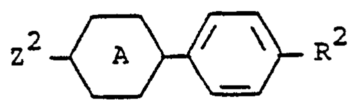

(III)

wherein one of the groups $Z^1$ and $Z^2$ denotes the carboxyl group and the other denotes the hydroxy group and $R^1$, $R^2$ and ring A have the significances given in claim 1, or reactive derivatives of these compounds, or

b) for the manufacture of the compounds of formula I in which X signifies the group —$CH_2CH_2$—, catalytically hydrogenating a compound of the general formula

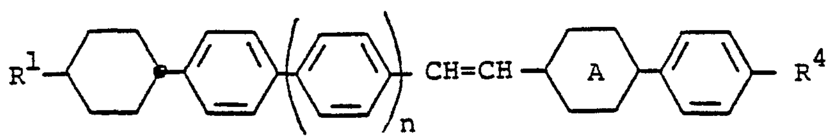

(IV)

wherein $R^4$ represents cyano or p-cyanophenyl and $R^1$, n and ring A have the significances given in claim 1.

9. The use of the compounds of formula I defined in claim 1 for electro-optical purposes.

10. The use of the compounds of formula I defined in claim 1 in gas chromatography.

**Claims** (for the Contracting State AT)

1. A process for the manufacture of the compounds of the general formula

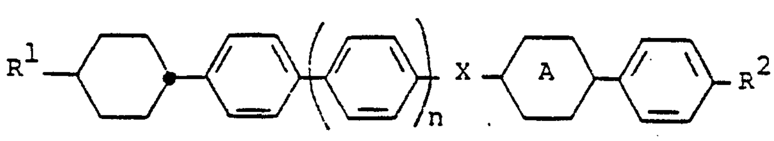

(I)

wherein n stands for the number 1 and X stands for the group —COO—, —OOC— or —$CH_2CH_2$—, or n stands for the number 0 and X stands for the group —$CH_2CH_2$— ; ring A denotes p-phenylene or trans-

1,4-cyclohexylene ; $R^2$ represents cyano, p-cyanophenyl or, insofar as X stands for —COO— or —OOC—, also p-$R^3$-phenyl or trans-4-$R^3$-cyclohexyl ; and $R^1$ and $R^3$ signify straight-chain $C_1$-$C_{10}$-alkyl, characterized by

a) for the manufacture of the compounds of formula I in which X signifies the group —COO— or —OOC—, esterifying a compound of the general formula

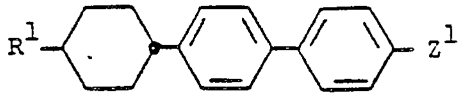

(II)

and a compound of the general formula

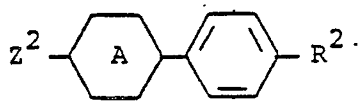

(III)

wherein one of the groups $Z^1$ and $Z^2$ denotes the carboxyl group and the other denotes the hydroxy group and $R^1$, $R^2$ and ring A have the above significances, or reactive derivatives of these compounds, or

b) for the manufacture of the compounds of formula I in which X signifies the group $—CH_2CH_2—$, catalytically hydrogenating a compound of the general formula

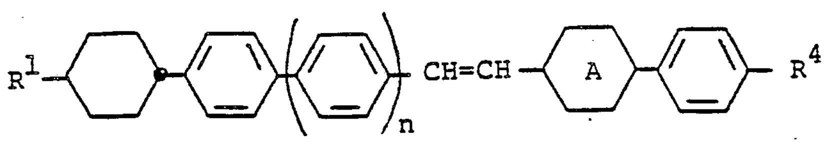

(IV)

wherein $R^4$ represents cyano or p-cyanophenyl and $R^1$, n and ring A have the above significances.

2. A process according to claim 1, characterized in that n stands for the number 1.

3. A process according to claim 1 or 2, characterized in that ring A denotes trans-1,4-cyclohexylene and $R^2$ denotes cyano, p-cyanophenyl or p-$R^3$-phenyl or ring A denotes p-phenylene and $R^2$ denotes trans-4-$R^3$-cyclohexyl.

4. A process according to any one of claims 1 to 3, characterized in that X stands for the group —COO—.

5. A process according to any one of claims 1 to 3, characterized in that X stands for the group $—CH_2CH_2—$.

6. A process according to any one of claims 1 to 4, characterized in that $R^2$ represents p-$R^3$-phenyl or trans-4-$R^3$-cyclohexyl and $R^3$ signifies propyl, butyl or pentyl.

7. A liquid crystalline mixture with at least 2 components, characterized in that at least one component is a compound of formula I defined in claim 1.

8. The use of the compounds of formula I defined in claim 1 for electro-optical purposes.

9. The use of the compounds of formula I defined in claim 1 in gas chromatography.

**Revendications** (pour les Etats contractants : BE, CH, DE, FR, GB, IT, LI, NL)

1. Composés de formule générale

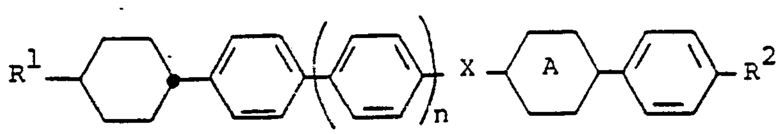

(I)

où n représente le nombre 1 et X représente le groupe —COO—, —OOC— ou $—CH_2CH_2—$, ou n représente le nombre 0 et X le groupe $—CH_2CH_2—$ ; le noyau A représente le p-phénylène ou le trans-1,4-cyclohexylène ; $R^2$ représente un cyano, un p-cyanophényle ou encore, si X représente —COO— ou —OOC—, un p-$R^3$-phényle ou un trans-4-$R^3$-cyclohexyle ; et $R^1$ et $R^3$ représentent un alcoyle en $C_1$ à $C_{10}$ à chaîne droite.

2. Composés selon la revendication 1, caractérisés en ce que n représente le nombre 1.

3. Composés selon la revendication 1 ou 2, caractérisés en ce que le noyau A représente le trans-1,4-cyclohexylène et $R^2$ un cyano, p-cyanophényle ou p-$R^3$-phényle ou le noyau A le p-phénylène et $R^2$ le trans-4-$R^3$-cyclohexyle.

4. Composés selon l'une des revendications 1 à 3, caractérisés en ce que X représente le groupe —COO—.

5. Composés selon l'une des revendications 1 à 3, caractérisés en ce que X représente le groupe —$CH_2CH_2$—.

6. Composés selon l'une des revendications 1 à 4, caractérisés en ce que $R^2$ représente un p-$R^3$-phényle ou trans-4-$R^3$-cyclohexyle et $R^3$ un propyle, butyle ou pentyle.

7. Mélange à cristaux liquides ayant au moins 2 composants, caractérisé en ce qu'au moins un composant est un composé de la formule I définie dans la revendication 1.

8. Procédé de préparation des composés de la formule I définie dans la revendication 1, caractérisé en ce que

a) pour préparer les composés de formule I où X représente le groupe —COO— ou —OOC—, on estérifie un composé de formule générale

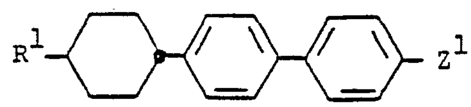

(II)

et un composé de formule générale

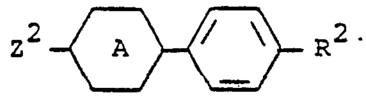

(III)

où l'un des groupes $Z^1$ et $Z^2$ représente le groupe carboxyle et l'autre le groupe hydroxy et $R^1$, $R^2$ et le noyau A ont la signification donnée dans la revendication 1, ou des dérivés réactifs de ces composés, ou

b) pour préparer les composés de formule I où X représente le groupe —$CH_2CH_2$—, on hydrogène catalytiquement un composé de formule générale

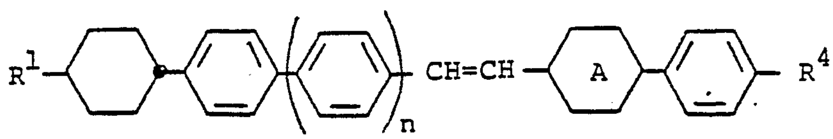

(IV)

où $R^4$ représente un cyano ou un p-cyanophényle et $R^1$, n et le noyau A ont les significations données dans la revendication 1.

9. Application des composés de formule I définie dans la revendication 1 pour des buts électro-optiques.

10. Application des composés de la formule I définie dans la revendication 1 dans la chromatographie en phase gazeuse.

**Revendications** (pour l'Etat contractant AT)

1. Procédé de préparation des composés de formule générale

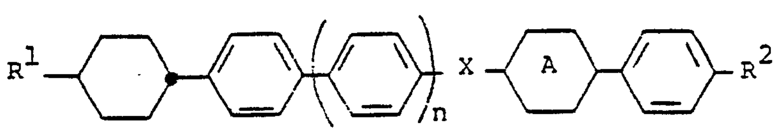

(I)

où n représente le nombre 1 et X le groupe —COO—, —OOC— ou —$CH_2CH_2$—, ou n représente le nombre 0 et X le groupe —$CH_2CH_2$— ; le noyau A représente le p-phénylène ou le trans-1,4-cyclohexylène ; $R^2$ représente un cyano, p-cyanophényle ou encore, si X représente —COO— ou —OOC—, p-$R^3$-phényle ou trans-4-$R^3$-cyclohexyle ; et $R^1$ et $R^3$ représentent un alcoyle en $C_1$ à $C_{10}$ à chaîne droite,
caractérisé en ce que

a) pour préparer les composés de formule I où X représente le groupe —COO— ou —OOC—, on estérifie un composé de formule générale

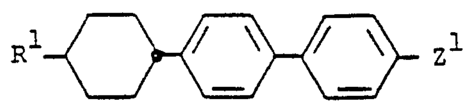

(II)

et un composé de formule générale

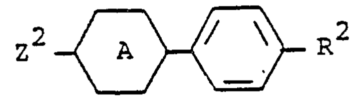

(III)

où l'un des groupes $R^1$ et $R^2$ représente le groupe carboxyle et l'autre le groupe hydroxy et $R^1$, $R^2$ et le noyau A ont les significations données ci-dessus,
ou des dérivés réactifs de ces composés, ou

b) pour préparer les composés de formule I où X représente le groupe $—CH_2CH_2—$, on hydrogène catalytiquement un composé de formule générale

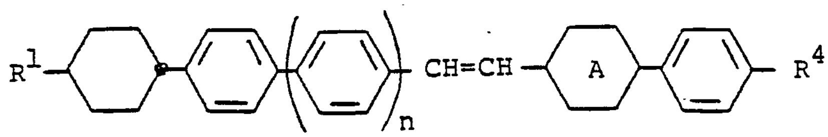

(IV)

où $R^4$ représente un cyano ou un p-cyanophényle et $R^1$, n et le noyau A ont les significations données ci-dessus.

2. Procédé selon la revendication 1, caractérisé en ce que n représente le nombre 1.

3. Procédé selon la revendication 1 ou 2, caractérisé en ce que le noyau A représente le trans-1,4-cyclohexylène et $R^2$ un cyano, p-cyanophényle ou p-$R^3$-phényle, ou le noyau A représente un p-phénylène et $R^2$ un trans-4-$R^3$-cyclohexyle.

4. Procédé selon l'une des revendications 1 à 3, caractérisé en ce que X représente le groupe —COO—.

5. Procédé selon l'une des revendications 1 à 3, caractérisé en ce que X représente le groupe $—CH_2CH_2—$.

6. Procédé selon l'une des revendications 1 à 4, caractérisé en ce que $R^2$ représente un p-$R^3$-phényle ou trans-4-$R^3$-cyclohexyle et $R^3$ représente un propyle, butyle ou pentyle.

7. Mélange à cristaux liquides ayant au moins 2 composants, caractérisé en ce qu'au moins un composant est un composé de formule I définie dans la revendication 1.

8. Application des composés de la formule I définie dans la revendication 1 pour des buts électro-optiques.

9. Application des composés de formule I définie dans la revendication 1 dans la chromatographie en phase gazeuse.

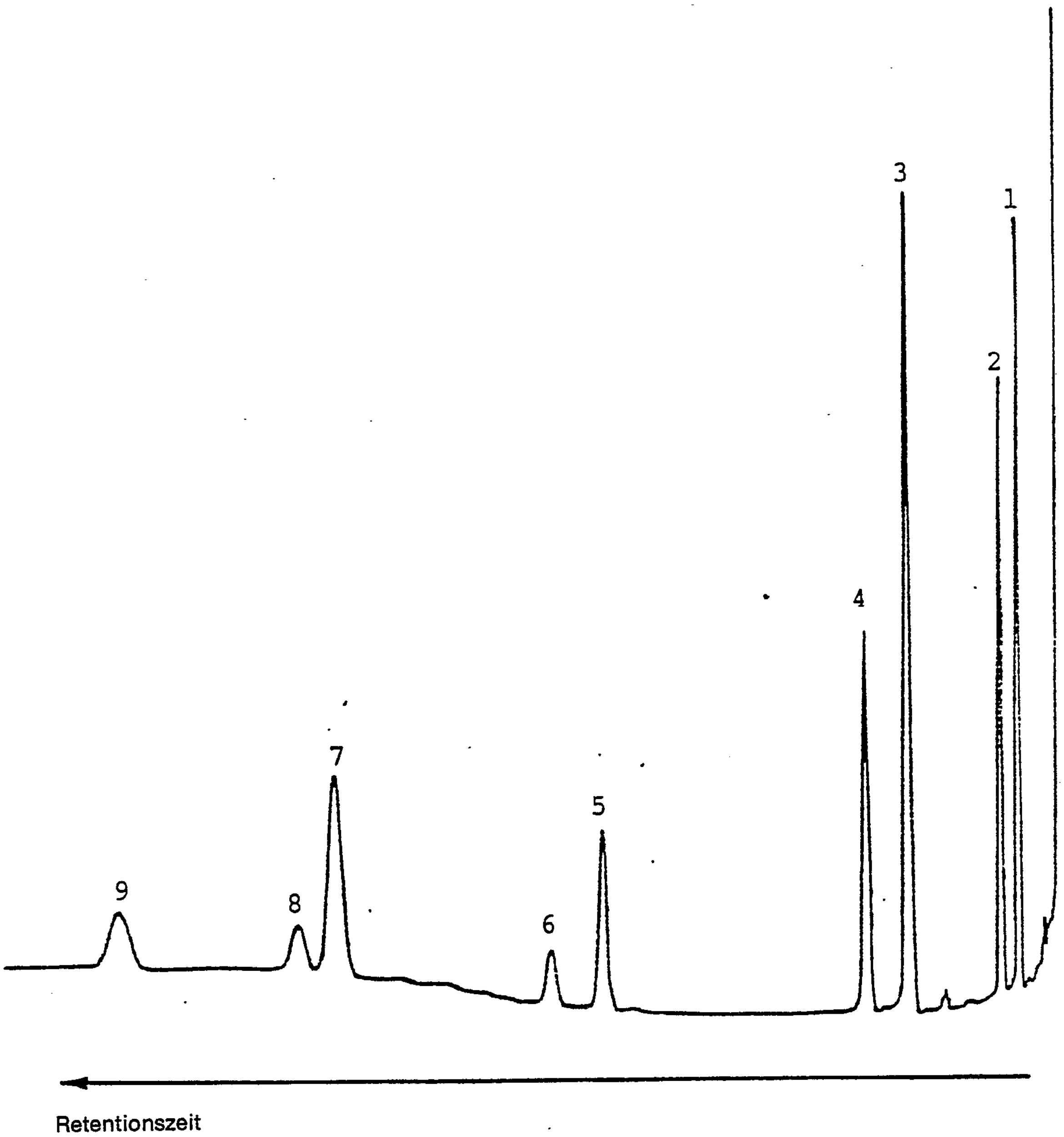


Figur 1 : Trennung aromatischer Kohlenwasserstoffe (gemäss Beispiel 9)